# EUROPEAN PATENT SPECIFICATION

(11) **EP 1 639 138 B1**
(45) Date of publication and mention of the grant of the patent: **30.11.2011**
(21) Application number: 04763058.7
(22) Date of filing: 30.06.2004
(51) Int. Cl.: C12Q 1/68

(54) **SPECIFIC METHOD OF PROSTATE CANCER DETECTION BASED ON PCA3 GENE, AND KITS THEREFOR**
SPEZIFISCHES VERFAHREN ZUM NACHWEIS VON PROSTATAKREBS AUF GRUNDLAGE DES PCA3-GENS SOWIE KITS DAFÜR
METHODE SPECIFIQUE DE DETECTION DU CANCER DE LA PROSTATE BASEE SUR PCA3 ET TROUSSES ASSOCIEES

(30) Priority: 30.06.2003 CA 2432365
(43) Date of publication of application: 29.03.2006
(73) Proprietor: Stichting Katholieke Universiteit, The University Medical Centre Nijmegen, 6500 HB Nijmegen (NL)
(72) Inventor: SCHALKEN, Jack, A., NL-6524 LH Nijmegen (NL); VERHAEGH, Gerald, NL-6584 GY Molenhoek (NL); HESSELS, Daphne, NL-6581 HJ Malden (NL); SMIT, Frank, NL-6546 RN Nijmegen (NL)
(74) Representative: Vossius & Partner
(86) International application number: PCT/EP2004/007124
(87) International publication number: WO 2005/003387

(56) References cited:
- HESSELS D ET AL: "DD3PCA3-BASED MOLECULAR URINE ANALYSIS FOR THE DIAGNOSIS OF PROSTATE CANCER" EUROPEAN UROLOGY, S. KARGER AG., BASEL, CH, vol. 44, no. 1, 28 March 2003 (2003-03-28), pages 8-16, XP009032062 ISSN: 0302-2838
- BUSSEMAKERS M J G ET AL: "DD3: a new prostate-specific gene, highly overexpressed in prostate cancer" CANCER RESEARCH, AMERICAN ASSOCIATION FOR CANCER RESEARCH, BALTIMORE, MD, US, vol. 59, no. 23, 1 December 1999 (1999-12-01), pages 5975-5979, XP002284051 ISSN: 0008-5472
- KOK DE J B ET AL: "DD3PCA3, A VERY SENSITIVE AND SPECIFIC MARKER TO DETECT PROSTATE TUMORS" CANCER RESEARCH, AMERICAN ASSOCIATION FOR CANCER RESEARCH, BALTIMORE, MD, US, vol. 62, no. 9, 1 May 2002 (2002-05-01), pages 2695-2698, XP001189400 ISSN: 0008-5472
- DATABASE NCBI 21 August 2000 (2000-08-21), BUSSEMAKERS ET AL.: "Homo sapiens non-coding RNA DD3 gene, exons 2, 3, and" XP002308338 Database accession no. AF103908
- DATABASE NCBI 14 August 2000 (2000-08-14), BUSSEMAKERS ET AL.: "Homo sapiens non-coding RNA DD3 sequence" XP002308339 Database accession no. AF103907
- GANDINI O ET AL: "IS DD3 A NEW PROSTATE-SPECIFIC GENE?" ANTICANCER RESEARCH, HELENIC ANTICANCER INSTITUTE, ATHENS,, GR, vol. 23, no. 1A, January 2003 (2003-01), pages 305-308, XP009032063 ISSN: 0250-7005
- VERHAEGH GERALD W; DE KOK JACQUES B; HESSELS DAPHNE; SMIT FRANK; SCHALKEN JACK A: "Correspondence re: J. B. de Kok et al., DD3, a very sensitive and specific marker to detect prostate tumors. Cancer Res., 62, 2695-2698, 2002. Reply." CANCER RESEARCH, vol. 63, 1 August 2003 (2003-08-01), pages 4748-4749,

### SEQUENCE LISTING

(1) GENERAL INFORMATION:
   (i) APPLICANT: Nijmegen university
   (ii) TITLE OF INVENTION: SPECIFIC METHOD OF PROSTATE CANCER DETECTION BASED ON PCA3, AND KITS THEREFOR
   (iii) NUMBER OF SEQUENCES: 2
(2) INFORMATION FOR SEQ ID NO: 1:
   (i) SEQUENCE CHARACTERISTICS:
      (A) LENGTH: 2037 base pairs
      (B) TYPE: nucleic acid
      (C) STRANDEDNESS: double
      (D) TOPOLOGY: linear
   (ii) MOLECULE TYPE: cDNA
   (xi) SEQUENCE DESCRIPTION: SEQ ID NO:1: 
AGAAGCTGGC ATCAGAAAAA CAGAGGGGAG ATTTGTGTGG CTGCAGCCGA GGGAGACCAG 60

GAAGATCTGC ATGGTGGAAA GGACCTGATG ATACAGAGGA ATTACAACAC ATATACTTAG 120

TGTTTCAATG AACACCAAGA TAAATAAGTG AAGAGCTAGT CCGCTGTGAG TCTCCTCAGT 180

GACACAGGGC TGGATCACCA TCGACGGCAC TTTCTGAGTA CTCAGTGCAG CAAAGAAAGA 240

CTACAGACAT CTCAATGGCA GGGGTGAGAA ATAAGAAAGG CTGCTGACTT TACCATCTGA 300

GGCCACACAT CTGCTGAAAT GGAGATAATT AACATCACTA GAAACAGCAA GATGACAATA 360

TAATGTCTAA GTAGTGAC ATGTTTTG CACATTTCC AGCCCC TTT AAA TAT 411

CCA CAC ACA CAG GAA GCA CAA AAG GAA GCA CAG AGA TCC CTG GGA GAA 459

ATG CCC GGC CGC CAT CTT GGG TCA TCG ATG AGC CTC GCC CTG TGC CTG 507

GTC CCG CTT GTG AGG GAA GGA CATTAGAAAATGA ATTGATGTGT TCCTTAAAGG 561

ATGGGCAGGA AAACAGATCC TGTTGTGGAT ATTTATTTGA ACGGGATTAC AGATTTGAAA 621

TGAAGTCACA AAGTGAGCAT TACCAATGAG AGGAAAACAG ACGAGAAAAT CTTGATGGCT 681

TCACAAGACA TGCAACAAAC AAAATGGAAT ACTGTGATGA CATGAGGCAG CCAAGCTGGG 741

GAGGAGATAA CCACGGGGCA GAGGGTCAGG ATTCTGGCCC TGCTGCCTAA ACTGTGCGTT 801

CATAACCAAA TCATTTCATA TTTCTAACCC TCAAAACAAA GCTGTTGTAA TATCTGATCT 861

CTACGGTTCC TTCTGGCCCC AACATTCTCC ATATATCCAG CCACACTCAT TTTTAATATT 921

TAGTTCCCAG ATCTGTACTG TGACCTTTCT ACACTGTAGA ATAACATTAC TCATTTTGTT 981
 
CAAAGACCCT TCGTGTTGCT GCCTAATATG TAGCTGACTG TTTTTCCTAA GGAGTGTTCT 1041

GGCCCAGGGG ATCTGTGAAC AGGCTGGGAA GCATCTCAAG ATCTTTCCAG GGTTATACTT 1101

ACTAGCACAC AGCATGATCA TTACGGAGTG AATTATCTAA TCAACATCAT CCTCAGTGTC 1161

TTTGCCCATA CTGAAATTCA TTTCCCACTT TTGTGCCCAT TCTCAAGACC TCAAAATGTC 1221

ATTCCATTAA TATCACAGGA TTAACTTTTT TTTTTAACCT GGAAGAATTC AATGTTACAT 1281

GCAGCTATGG GAATTTAATT ACATATTTTG TTTTCCAGTG CAAAGATGAC TAAGTCCTTT 1341

ATCCCTCCCC TTTGTTTGAT TTTTTTTCCA GTATAAAGTT AAAATGCTTA GCCTTGTACT 1401

GAGGCTGTAT ACAGCACAGC CTCTCCCCAT CCCTCCAGCC TTATCTGTCA TCACCATCAA 1461

CCCCTCCCAT NYSACCTAAA CAAAATCTAA CTTGTAATTC CTTGAACATG TCAGGNCATA 1521

CATTRTTCCT TCTGCCTGAG AAGCTCTTCC TTGTCTCTTA ANTCTAGAAT GATGTAAAGT 1581

TTTGAATAAG TTGACTATCT TACTTCATGC AAAGAAGGGA CACATATGAG ATTCATCATC 1641

ACATGAGACA GCAAATACTA AAAGTGTAAT TTGATTATAA GAGTTTAGAT AAATATATGA 1701

AATGCAAGAK CCACAGAGGG AATGTTTATG GGGCACGTTT GTAAGCCTGG GATGTGAAGM 1761

AAAGGCAGGG AACCTCATAG TATCTTATAT AATATACTTC ATTTCTCTAT CTCTATCACA 1821

ATATCCAACA AGCTTTTCAC AGAATTCATG CAGTGCAAAT CCCCAAAGGT AACCTTTATC 1881

CATTTCATGG TGAGTGCGCT TTAGAATTTT GGCAAATCAT ACTGGTCACT TATCTCAAGT 1941

TTGAGATGTG TTTGTCCTTG TAGTTAATTG AAAGAAATAG GGCACTCTTG TGAGCCACTT 2001

TAGGGTTCAC TCCTGGCAAT AAAGAATTTA CAAAGA 2037
(2) INFORMATION FOR SEQ ID NO: 2:
   (i) SEQUENCE CHARACTERISTICS:
      (A) LENGTH: 3582 base pairs
      (B) TYPE: nucleic acid
      (C) STRANDEDNESS: double
      (D) TOPOLOGY: linear
   (ii) MOLECULE TYPE: cDNA
   (xi) SEQUENCE DESCRIPTION: SEQ ID NO: 2:

ACAGAAGAAA TAGCAAGTGC CGAGAAGCTG GCATCAGAAA AACAGAGGGG AGATTTGTGT 60

GGCTGCAGCC GAGGGAGACC AGGAAGATCT GCATGGTGGG AAGGACCTGA TGATACAGAG 120

GAATTACAAC ACATATACTT AGTGTTTCAA TGAACACCAA GATAAATAAG TGAAGAGCTA 180

GTCCGCTGTG AGTCTCCTCA GTGACACAGG GCTGGATCAC CATCGACGGC ACTTTCTGAG 240

TACTCAGTGC AGCAAAGAAA GACTACAGAC ATCTCAATGG CAGGGGTGAG AAATAAGAAA 300

GGCTGCTGAC TTTACCATCT GAGGCCACAC ATCTGCTGAA ATGGAGATAA TTAACATCAC 360

TAGAAACAGC AAGATGACAA TATAATGTCT AAGTAGTGAC ATGTTTTG CACATT 415

TCC AGC CCC TTT AAA TAT CCA CAC ACA CAG GAA GCA CAA AAG GAA GCA 463

CAG AGA TCC CTG GGA GAA ATG CCC GGC CGC CAT CTT GGG TCA TCG ATG 511

AGC CTC GCC CTG TGC CTG GTC CCG CTT GTG AGG GAA GGA CAT 553

TAGAAAATGA ATTGATGTGT TCCTTAAAGG ATGGGCAGGA AAACAGATCC TGTTGTGGAT 613

ATTTATTTGA ACGGGATTAC AGATTTGAAA TGAAGTCACA AAGTGAGCAT TACCAATGAG 673

AGGAAAACAG ACGAGAAAAT CTTGATGGCT TCACAAGACA TGCAACAAAC AAAATGGAAT 733

ACTGTGATGA CATGAGGCAG CCAAGCTGGG GAGGAGATAA CCACGGGGCA GAGGGTCAGG 793

ATTCTGGCCC TGCTGCCTAA ACTGTGCGTT CATAACCAAA TCATTTCATA TTTCTAACCC 853

TCAAAACAAA GCTGTTGTAA TATCTGATCT CTACGGTTCC TTCTGGGCCC AACATTCTCC 913

ATATATCCAG CCACACTCAT TTTTAATATT TAGTTCCCAG ATCTGTACTG TGACCTTTCT 973

ACACTGTAGA ATAACATTAC TCATTTTGTT CAAAGACCCT TCGTGTTGCT GCCTAATATG 1033

TAGCTGACTG TTTTTCCTAA GGAGTGTTCT GGCCCAGGGG ATCTGTGAAC AGGCTGGGAA 1093

GCATCTCAAG ATCTTTCCAG GGTTATACTT ACTAGCACAC AGCATGATCA TTACGGAGTG 1153

AATTATCTAA TCAACATCAT CCTCAGTGTC TTTGCCCATA CTGAAATTCA TTTCCCACTT 1213

TTGTGCCCAT TCTCAAGACC TCAAAATGTC ATTCCATTAA TATCACAGGA TTAACTTTTT 1273

TTTTTAACCT GGAAGAATTC AATGTTACAT GCAGCTATGG GAATTTAATT ACATATTTTG 1333

TTTTCCAGTG CAAAGATGAC TAAGTCCTTT ATCCCTCCCC TTTGTTTGAT TTTTTTTCCA 1393

GTATAAAGTT AAAATGCTTA GCCTTGTACT GAGGCTGTAT ACAGCACAGC CTCTCCCCAT 1453

CCCTCCAGCC TTATCTGTCA TCACCATCAA CCCCTCCCAT ACCACCTAAA CAAAATCTAA 1513

CTTGTAATTC CTTGAACATG TCAGGACATA CATTATTCCT TCTGCCTGAG AAGCTCTTCC 1573

TTGTCTCTTA AATCTAGAAT GATGTAAAGT TTTGAATAAG TTGACTATCT TACTTCATGC 1633

AAAGAAGGGA CACATATGAG ATTCATCATC ACATGAGACA GCAAATACTA AAAGTGTAAT 1693

TTGATTATAA GAGTTTAGAT AAATATATGA AATGCAAGAG CCACAGAGGG AATGTTTATG 1753

GGGCACGTTT GTAAGCCTGG GATGTGAAGC AAAGGCAGGG AACCTCATAG TATCTTATAT 1813

AATATACTTC ATTTCTCTAT CTCTATCACA ATATCCAACA AGCTTTTCAC AGAATTCATG 1873

CAGTGCAAAT CCCCAAAGGT AACCTTTATC CATTTCATGG TGAGTGCGCT TTAGAATTTT 1933

GGCAAATCAT ACTGGTCACT TATCTCAACT TTGAGATGTG TTTGTCCTTG TAGTTAATTG 1993

AAAGAAATAG GGCACTCTTG TGAGCCACTT TAGGGTTCAC TCCTGGCAAT AAAGAATTTA 2053

CAAAGAGCTA CTCAGGACCA GTTGTTAAGA GCTCTGTGTG TGTGTGTGTG TGTGTGTGAG 2113

TGTACATGCC AAAGTGTGCC TCTCTCTCTT GACCCATTAT TTCAGACTTA AAACAAGCAT 2173

GTTTTCAAAT GGCACTATGA GCTGCCAATG ATGTATCACC ACCATATCTC ATTATTCTCC 2233

AGTAAATGTG ATAATAATGT CATCTGTTAA CATAAAAAAA GTTTGACTTC ACAAAAGCAG 2293

CTGGAAATGG ACAACCACAA TATGCATAAA TCTAACTCCT ACCATCAGCT ACACACTGCT 2353

TGACATATAT TGTTAGAAGC ACCTCGCATT TGTGGGTTCT CTTAAGCAAA ATACTTGCAT 2413

TAGGTCTCAG CTGGGGCTGT GCATCAGGCG GTTTGAGAAA TATTCAATTC TCAGCAGAAG 2473

CCAGAATTTG AATTCCCTCA TCTTTTAGGA ATCATTTACC AGGTTTGGAG AGGATTCAGA 2533

CAGCTCAGGT GCTTTCACTA ATGTCTCTGA ACTTCTGTCC CTCTTTGTGT TCATGGATAG 2593

TCCAATAAAT AATGTTATCT TTGAACTGAT GCTCATAGGA GAGAATATAA GAACTCTGAG 2653

TGATATCAAC ATTAGGGATT CAAAGAAATA TTAGATTTAA GCTCACACTG GTCAAAAGGA 2713

ACCAAGATAC AAAGAACTCT GAGCTGTCAT CGTCCCCATC TCTGTGAGCC ACAACCAACA 2773

GCAGGACCCA ACGCATGTCT GAGATCCTTA AATCAAGGAA ACCAGTGTCA TGAGTTGAAT 2833

TCTCCTATTA TGGATGCTAG CTTCTGGCCA TCTCTGGCTC TCCTCTTGAC ACATATTAGC 2893

TTCTAGCCTT TGCTTCCACG ACTTTTATCT TTTCTCCAAC ACATCGCTTA CCAATCCTCT 2953

CTCTGCTCTG TTGCTTTGGA CTTCCCCACA AGAATTTCAA CGACTCTCAA GTCTTTTCTT 3013

CCATCCCCAC CACTAACCTG AATTGCCTAG ACCCTTATTT TTATTAATTT CCAATAGATG 3073

CTGCCTATGG GCTAATATTG CTTTAGATGA ACATTAGATA TTTAAAGTCT AAGAGGTTCA 3133

AAATCCAACT CATTATCTTC TCTTTCTTTC ACCTCCCCTG CTCCTCTCCC TATATTACTG 3193

ATTGACTGAA CAGGATGGTC CCCAAGATGC CAGTCAAATG AGAAACCCAG TGGCTCCTTG 3253

TGGATCATGC ATGCAAGACT GCTGAAGCCA GAGGATGACT GATTACGCCT CATGGGTGGA 3313

GGGGACCACT CCTGGGCCTT CGTGATTGTC AGGAGCAAGA CCTGAGATGC TCCCTGCCTT 3373

CAGTGTCCTC TGCATCTCCC CTTTCTAATG AAGATCCATA GAATTTGCTA CATTTGAGAA 3433

TTCCAATTAG GAACTCACAT GTTTTATCTG CCCTATCAAT TTTTTAAACT TGCTGAAAAT 3493

TAAGTTTTTT CAAAATCTGT CCTTGTAAAT TACTTTTTCT TACAGTGTCT TGGCATACTA 3553

TATCAACTTT GATTCTTTGT TACAACTTT 3582

## Description

### FIELD OF THIS INVENTION

The present invention relates, in general, to prostate cancer. More specifically, the present invention relates to a method to diagnose prostate cancer in a patient by detecting a PCA3 sequence, and more particularly a PCA3 RNA, the PCA3 sequence detected in a sample from the patient being specifically associated with prostate cancer. The invention also relates to kits containing nucleic acid primers and nucleic acid probes to diagnose, assess, or prognose a human afflicted with prostate cancer.

### BACKGROUND OF THE INVENTION

Over the last decade, cancer of the prostate has become the most commonly diagnosed malignancy among men and the second leading cause of male cancer deaths in the western population, following lung cancer.

Early detection and treatment of prostate cancer before it has spread from the prostate gland reduces the mortality of the disease. This is particularly true for younger men who are at greater risk of dying from this pernicious but slowly growing malignancy. This realization has prompted increasing efforts for early diagnosis and treatment. Indeed, the American Cancer Society and American Urological Association recommend that male population at large undergo annual screening for prostate cancer beginning at age 50. The recommended age for screening is lowered to 40 for men giving a family history of prostate cancer or other risk factors.

With this increasing focus on prostate cancer screening, more men than ever before are being routinely tested for prostate cancer. Not surprisingly, this practice has increased early detection of onset of the disease, as reflected by an apparent increase in the incidence of prostate cancer and decrease in the apparent average age of diagnosis. The clinical hope is that earlier detection of prostate cancer before it metastasizes will reduce the overall mortality rate. Healthcare payers look for early screening and detection to translate into a reduction in the healthcare burden, as early treatment can be less radical, more successful and therefore provided at a lower cost per treated patient The key to accomplishing this goal remains providing better differential diagnostic tools.

Screening for prostate cancer now involves both palpation of the prostate by digital rectal examination and assay of plasma levels of prostate specific antigen (PSA). PSA is a serine protease produced by the prostatic epithelium that is normally secreted in the seminal fluid to liquefy it. Disruption of the anatomic integrity of the prostate gland can compromise the cellular barriers that normally restrict PSA to within the duct system of the prostate, allowing it to disperse into blood or urine. A number of conditions can result in leakage of PSA into the blood. They include inflammation of the prostate, urinary retention, prostatic infection, benign prostatic hyperplasia, and prostate cancer. Physical manipulation of the prostate can also increase serum PSA levels, but a mild stimulus, such as digital rectal examination (DRE), does not normally increase serum PSA. It is therefore not surprising that screening of serum PSA as an indicator of prostate cancer is not absolutely predictive.

Despite the fact that measure of blood PSA levels can results from a variety of different causes, it is nonetheless the basis for primary screening for prostate cancer. Measurement of total PSA (tPSA) as a diagnostic assay to predict prostate cancer has been in use since 1991. Levels of 4 ng/ml or greater in blood serum are considered abnormal and predictive of prostate cancer. However, the sensitivity of such elevated tPSA levels is only 79%; thus leaving 21% of patients with prostate cancer undetected. The specificity for all tPSA values of 4 ng/ml or greater is very poor. In addition, estimates of specificity for tPSA levels > 4.0 ng/ml are reported to be in the rage of 20% to 59%, averaging around 33%. The vast majority of false positives are ultimately shown to be benign prostatic hyperplasia. The specificity is lowest for modestly elevated tPSA, in the low so-called gray zone of 4 to 10ng/ml. This low level of specificity results in additional more invasive and costly diagnostic procedures, such as transrectal ultrasounds and prostate biopsies. Such tests when unnecessary are also very traumatic for the patient. The psychological impact of being diagnosed as positive until proven as a false positive should not be understated either.

Because of the shortcomings of tPSA, research has been focused on attempting to develop PSA derivatives to increase the sensitivity and specificity of this general diagnostic approach.

One modification is free PSA (fPSA), which was FDA approved in 1998. PSA in serum can be found either in an unbound form or complexed with circulating protease inhibitors, most commonly with alpha-1-antitrypsin (ACT). Clinicians have shown that the proportion of PSA bound to ACT was significantly higher in men with prostate cancer than in unaffected men or those with benign prostate hypertrophy (BPH). As a guideline, if 25% or less of total PSA is free, this is an indicator of possible prostate cancer. The tPSA assay was approved for use in men with tPSA's for 4 to 10ng/ml. Thus, the fPSA assay was positioned to improve the specificity over that of tPSA alone. However, the predictivity of the fPSA test is not as good in people with really low or really high tPSA levels. Very low tPSA, regardless of measured fPSA, is predictive of not having cancer, while the converse is true with very high tPSA levels. The diagnostic usefulness of fPSA is relatively limited as it can be associated with either BPH or prostate cancer. The use of fPSA in combination with tPSA has been shown to reduce the number of unnecessary biopsies by about 20%.

Clearly, prostate biopsy is the gold standard for confirming prostate cancer. However, even a biopsy is not always 100% certain. The standard is the sextant biopsy where tissue sample collection is guided by transrectal ultrasound. Often the six samples do not detect the cancer and either a second biopsy procedure or more than six samples are required.

Despite the improvements to prostate cancer screening that have come along in the last ten years, there remains a large unmet need in diagnostic sensitivity and specificity, even when these tools are used in combination. Coupling this with the large incidence of prostate cancer and the need for early, accurate detection, the potential of a true differential diagnostic tool is very significant.

A new prostate cancer marker, PCA3, was discovered a few years ago by differential display analysis intended to highlight genes associated with prostate cancer development (USN 09/402,713; 09/675,650; 09/996,953; 60/445,436; WO 98/45420; and WO 01/23550). PCA3 is located on chromosome 9 and composed of four exons. It encodes at least four different transcripts which are generated by alternative splicing and polyadenylation. By RT-PCR analysis, PCA3 expression was found to be limited to the prostate and absent in all other tissues, including testis, ovary, breast and bladder. Northern blot analysis showed that PCA3 is highly expressed in the vast majority of prostate cancers examined (47 out of 50) whereas no or very low expression is detected in benign prostate hyperplasia or normal prostate cells from the same patients [Cancer Res., 1999. Dec 1:59(23):5975-9]. Moreover, a recent study comparing the clinical performance of RNA telomerase RT and RNA PCA3-detection in the case of prostate cancer showed that the PCA3 gene can be considered as a better marker [cancer Res., 2002. May 1;62(9):2695-8].

The PCA3 gene is composed of 4 exons (e1-e4) and 3 introns (i1-i3). While PCA3 appears to be recognized as the best prostate-cancer marker ever identified, this specificity has been contested in the literature. For example, Gandini et al. 2003, claim that the prostate-specific expression of PCA3 is restricted to that of exon 4 of the PCA3 gene. Moreover, use of PCA3 as a prostate-cancer marker has also been hampered by an inability to differentiate prostate cancer specific PCA3 and PCA3 nucleic acids expressed in non-prostate tissues [Bussemakers et al., 1999; de Kok et al., 2002; and Hessels et a)., 2003].

There thus remains a need to clarify the issue of the specificity of the PCA3 marker and provide tools that will specifically identify PCA3 sequences associated with prostate cancer.

The present invention seeks to meet these and other needs.

In view of the fact that advanced prostate cancer remains a life threatening disease reaching a very significant proportion of the male population, there remains a need to provide the most specific, selective, and rapid prostate cancer detection methods and kits.

The present invention seeks to meet these and other needs.

Moreover, use of PCA3 as a prostate-cancer marker has also been hampered by an inability to differentiate prostate cancer specific PCA3 and PCA3 nucleic acids expressed in non-prostate tissues [Bussemakers et al., 1999; de Kok et al., 2002; and Hessels et al., 2003].

### SUMMARY OF THE INVENTION

The present invention relates to diagnostic methods and kits which detect prostate cancer in a more specific and selective fashion than the methods and kits of the prior art.

The methods of the invention can be performed in vitro, ex vivo: or in vivo.

One aim of this invention is to describe a method to detect prostate cancer in a patient and especially from a urine sample by detecting PCA3 RNA which is associated with prostate cancer.

The invention describes an in vitro method for diagnosing or prognosing prostate cancer in a patient comprising amplifying a spliced prostate cancer specific PCA3 RNA which lacks at least one intron from a urine sample of a patient using a pair of primers, wherein one primer is specific for an exon-exon junction of PCA3, and detecting an amplification product derived therefrom, wherein said amplification product is associated with the presence of prostate cancer or predisposition thereto in said patient.

In one particular embodiment, the PCA3 nucleic acid which is identified is associated with a malignant prostatic state and not with a non-malignant prostatic state. In one such particular embodiment, the PCA3 nucleic acid is PCA3 mRNA. As described herein, the sample is any sample from a patient containing a prostatic cell or nucleic acid therefrom in sufficient quantity to allow amplification thereof, and detection thereof. In a particular embodiment, the PCA3 nucleic acid detected is a PCA3 mRNA associated with prostatic cancer and the sample is a urine sample.

In one particular embodiment, the present invention relates to a method to assess prostate cancer in a patient, by detecting in a urine sample , the presence of PCA3 RNA which does not contain an intron. In an embodiment of the present invention, the PCR3 RNA which is detected is a spliced RNA which lacks an intron (i3) between exons 3 and 4. In another yet particular embodiment of the present invention, the PCA3 RNA which is detected is a spliced RNA which lacks introns between exon 1 and exon 3. In a particularly preferred embodiment of the present invention, the PCA3 RNA which is detected is a spliced RNA which lacks introns between exon 1 and exon 4. In a particularly preferred embodiment of the present invention, the PCA3 RNA which is detected is a spliced RNA which lacks at least one intron (e.g. il between exons 1 and 2 and/or i3 between exons 3 and 4). In one embodiments, a PCA3 RNA lacking at least a first intron between exons 1 and 2 is specifically targeted and detected. In another embodiment, the PCA3 RNA, which is detected lacks an intron (i3) between exons 3 and 4. In yet another particular embodiment, the detected PCA3 sequence is an intron-less PCA3 RNA.

As described herein, the prostate cancer specific RNA encoded by the PCA3 gene (i.e. RNA) may be detected using an amplification method which amplifies a second prostate-specific sequence (which does not have to be associated with prostate cancer) also contained in the sample. A number of such second prostate-specific sequences can be used as long as they can serve as a control for prostate RNA. Non-limiting examples of such prostate-specific sequences include PSA (in this case a prostate-specific sequence often associated with prostate cancer), and other kallikrein family members. The amplification of the prostate-cancer specific PCA3 RNA, sequences and the prostate-specific sequences can be carried out simultaneously. The amplification of PCA3 and second prostate-specific sequence can be carried out in the same or in different reaction mixtures and simultaneously or not.

The invention provides a method of detecting prostate cancer-specific PCA3 RNA in a samples.

In one embodiment, the present invention relates to a method (and kits therefore; as regards the specific components of the kit, see below) to diagnose or prognose prostate cancer in a patient, comprising a detection of a spliced prostate cancer specific PCA3 RNA from the patient, which lacks at least one intron, in a urine sample of a patient, comprising at least one pair of primers which is designed to enable amplification of the PCA3 RNA, wherein one primer is specific for an exon-exon junction of the PCA3 and wherein the PCA3 RNA is associated with a presence of prostate cancer or predisposition thereto in the patient. In the present invention, the PCA3 RNA lacks at least one intron. Numerous sensitive methods of detection of nucleic acids are known, and can be adapted in accordance with the type of sample, the sensitivity of the detection, the amount of PCA3 nucleic acid, and the like. As described herein, the detection is effected with a probe designed from the sequences shown herein and may span an exon-exon junction of said PCA3 RNA. The sensitivity of the method (and kit therefor) may be increased by further performing an amplification of the PCA3 nucleic acid. Numerous amplification methods can be used. Many of them are described herein. In one particular embodiment, PCA3 nucleic acid, and preferably PCA3 RNA is amplified using a primer which hybridizes to an exonic sequence thereof. In yet a further embodiment, the detection of PCA3 RNA is a detection of an RNA lacking more than one intron. As described herein it is usually carried out, but not necessarily after an amplification, that one or more probe can be used to detect the PCA3 targets an exon-exon junction. Kits enabling such methods are also within the scope of the present invention. The permutations of priming through different exon-junctions, of probing one or more exon junction, or detecting PCA3 RNA lacking a certain intron, more than one, etc, is taught herein, as well as methods to increase sensitivity, to capture the nucleic acid, and the like, are all taught or exemplified herein.

The invention also provides kits for detecting the presence of spliced prostate cancer-specific PCA3 RNA which lacks at least one intron in a urine sample in a patient, comprising at least one pair of primers which is designed to enable amplification of the PCA3 RNA, wherein one primer is specific for an exon-exon junction of the PCA3. As described herein, the diagnostic kit may comprise at least a first container means containing a pair of primers which can amplify the above-described prostate cancer-specific PCA3 nucleic acid (e.g. RNA). The diagnostic kit may also comprise a first container containing a pair of primers which can amplify the above mentioned prostate specific PCA3 RNA and a second container means containing a pair of primers which can amplify the above-mentioned second prostate-specific sequence. As described herein, the diagnostic kit may also comprise a third container means contains a probe which specifically hybridizes to the PCA3 amplification product. The probe further increases the specificity of the method, by specifically hybridizing to a chosen exon-exon junction of PCA3. A fourth container may be included containing a probe for another region of PCA3 and/or for the second prostate-specific sequence. The kit may also comprise reagents to increase the sensitivity of the detection. The kit may also contain reagents enabling real-time amplification and detection.

The invention thus further provides a method of diagnosing the presence or predisposition to develop prostate cancer in a patient.

In another embodiment, the RNA encoded by the PCA3 gene is obtained from a cell contained in a voided urine sample from the patient.

As described herein, the RNA may be detected using an RNA amplification method. The RNA amplification method may be coupled to real-time detection of the amplified products using fluorescence specific probes. In one embodiment, the amplification method is PCR. The PCR may be real-time PCR or a related method enabling a detection in real-time of the amplified products:

The urine sample may be obtained after an attentive digital rectal examination (DRE). Of course, it should be understood that the present methods and kits could also be used on a urine sample obtained without digital rectal examination; or on other types of samples such as sperm, mixed urine and sperm (first urine sample following ejaculation), provided that the amplification method and/or detection method is sensitive enough to detect the targeted markers (PCA3 and when desired the second marker). Experiments showed that the methods and kits as described herein could also be performed with these types of samples, which include blood or serum.

Cells collected from the urine sample may be harvested and a total nucleic acid extraction is carried out

Total nucleic acid extraction may be carried out using a solid phase band method on silica beads as described by BOOM et aL Of course, it should be understood that numerous nucleic acid extraction methods exists and thus, that other methods could be used in accordance with the present invention. One non-limiting example is a phenol/chloroform extraction method. Other such methods are described in herein referenced textbook.

RNA encoded by a PCA3 gene may be detected by an in vitro RNA amplification method named Nucleic Acid based Amplification (NASBA). Of course other RNA amplification methods are known and the instant methods and kits are therefore not limited to NASBA. Non-limiting examples of such RNA amplification methods include polymerase chain reaction (PCR), transcriptase mediated amplification (TMA) and ligase chain reaction (LCR).

The amplified products may be detected in homogenous phase using a fluorescent probe using the Beacon approach. In another embodiment, the product is detected on solid phase using fluorescent or colorimetric method. It should be understood that numerous fluorescent, colorimetric or enzymatic methods could be used in accordance with the present invention to detect and/or quantify the targeted RNAs. Such fluorescent, colorimetric or enzymatic methods are well known in the art.

It should be understood by a person of ordinary skill that numerous statistical methods can be used in the context of the present invention to determine if the test is positive or negative.

Further objects and advantages of the present invention will be clear from the description that follows.

### DEFINITIONS

In the description that follows, a number of terms used in DNA technology are extensively utilized. In order to provide a clear and consistent understanding of the specification and claims, including the scope to be given such terms, the following definitions are provided.

Unless defined otherwise, the scientific and technological terms and nomenclature used herein have the same meaning as commonly understood by a person of ordinary skill to which this invention pertains. Commonly understood definitions of molecular biology terms can be found for example in Dictionary of Microbiology and Molecular Biology, 2nd ed. (Singleton et al., 1994, John Wiley & Sons, New York, NY), The Harper Collins Dictionary of Biology (Hale & Marham, 1991, Harper Perennial, New York, NY), Rieger et aL, Glossary of genetics: Classical and molecular, 5th edition, Springer-Verlag, New-York, 1991; Alberts et al., Molecular Biology of the Cell, 4th edition, Garland science, New-York, 2002; and, Lewin, Genes VII, Oxford University Press, New-York, 2000. Generally, the procedures of cell cultures, infection, molecular biology methods and the like are common methods used in the art. Such standard techniques can be found in reference manuals such as for example Sambrook et al. (2000, Molecular Cloning - A Laboratory Manual, Third Edition, Cold Spring Harbor Laboratories); and Ausubel et al. (1994, Current Protocols in Molecular Biology, John Wiley & Sons, New-York).

The use of the word "a" or "an" when used in conjunction with the term "comprising" in the claims and/or the specification may mean "one" but it is also consistent with the meaning of "one or more", "at least one", and "one or more than one".

Throughout this application, the term "about" is used to indicate that a value includes the standard deviation of error for the device or method being employed to determine the value. Routinely a 10% to 15% deviation preferably 10% is within the scope of the term "about".

Isolated Nucleic Acid Molecule. An "isolated nucleic acid molecule", as is generally understood and used herein, refers to a polymer of nucleotides, and includes but should not be limited to DNA and RNA. The "isolated" nucleic acid molecule is purified from its natural *in vivo* state.

DNA Segment. A DNA segment, as is generally understood and used herein, refers to a molecule comprising a linear stretch of nucleotides wherein the nucleotides are present in a sequence that can encode, through the genetic code, a molecule comprising a linear sequence of amino acid residues that is referred to as a protein, a protein fragment or a polypeptide.

Gene. A DNA sequence related to a single polypeptide chain or protein, and as used herein includes the 5' and 3' untranslated ends. The polypeptide can be encoded by a full-length sequence or any portion of the coding sequence, so long as the functional activity of the protein is retained.

Complementary DNA (cDNA). Recombinant nucleic acid molecules synthesized by reverse transcription of messenger RNA ("RNA").

Structural Gene. A DNA sequence that is transcribed into RNA that is then translated into a sequence of amino acids characteristic of a specific polypeptide.

Agarose Gel Electrophoresis. The most commonly used technique (though not the only one) for fractionating double strand DNA is agarose gel electrophoresis. The principle of this method is that DNA molecules migrate through the gel as though it were a sieve that retards the movement of the largest molecules to the greatest extent and the movement of the smallest molecules to the least extent. Note that the smaller the DNA fragment, the greater the mobility under electrophoresis in the agarose gel.

The DNA fragments fractionated by agarose gel electrophoresis can be visualized directly by a staining procedure if the number of fragments included in the pattern is small. In order to visualize a small subset of these fragments, a methodology referred to as the Southern hybridization procedure can be applied.

Southern Transfer Procedure. The purpose of the Southern transfer procedure (also referred to as blotting) is to physically transfer DNA fractionated by agarose gel electrophoresis onto a nitrocellulose filter paper or another appropriate surface or method, while retaining the relative positions of DNA fragments resulting from the fractionation procedure. The methodology used to accomplish the transfer from agarose gel to nitrocellulose involves drawing the DNA from the gel into the nitrocellulose paper by capillary action.

Nucleic Acid Hybridization. Nucleic acid hybridization depends on the principle that two single-stranded nucleic acid molecules that have complementary base sequences will reform the thermodynamically favored double-stranded structure if they are mixed under the proper conditions. The double-stranded structure will be formed between two complementary single-stranded nucleic acids even if one is immobilized on a nitrocellulose filter. In the Southern hybridization procedure, the latter situation occurs. As noted previously, the DNA of the individual to be tested is digested with a restriction endonuclease, fractionated by agarose gel electrophoresis, converted to the single-stranded form, and transferred to nitrocellulose paper, making it available for reannealing to the hybridization probe. Examples of hybridization conditions can be found in Ausubel, F.M. et al., Current protocols in Molecular Biology, John Wily & Sons, Inc., New York, NY (1989). A nitrocellulose filter is incubated overnight at 42°C with labeled probe in a solution containing 50% formamide, (or at 68°C without formamide) high salt (either 5x SSC[20X: 3M NaCl/0.3M trisodium citrate] or 5X SSPE [20X: 3.6M NaCl/0.2M NaH₂PO₄/0.02M EDTA, pH 7.7]), 5X Denhardt's solution, 1% SDS, and 100 µg/ml denatured salmon sperm DNA. This is followed by several washes in 0.2X SSC/0.1% SDS at a temperature selected based on the desired stringency: room temperature (low stringency), 42°C (moderate astringency) or 68°C (high stringency). The temperature selected is determined based on the melting temperature (Tm) of the DNA hybrid. Formamide can also be used in the washings and the temperature is adapted in accordance with the desired Tm.

Hybridization Probe. To visualize a particular DNA sequence in the Southern hybridization procedure (e.g. an amplification product), a-labeled DNA molecule or hybridization probe is reacted to the fractionated DNA bound to the nitrocellulose filter. The areas on the filter that carry DNA sequences complementary to the labeled DNA probe become labeled themselves as a consequence of the re-annealing reaction. The areas of the filter that exhibit such labeling are visualized. The hybridization probe is generally produced by molecular cloning of a specific DNA sequence. In one particular embodiment the probe spans the 3' region of a first exon and the 5' region of a second exon, such that such a probe can only detect the amplification product if the first exon and second exon have been spliced into a contiguous position (i.e. by removing an intervening intronic sequence). Knowing the sequences of the exon boundaries, as well as those of the different exons (see below), the numerous primers and probes which can be designed and used in the context of the present invention can be readily determined by a person of ordinary skill in the art to which the present invention pertains.

Oligonucleotide, Oligomer or oligo. A molecule comprised of two or more deoxyribonucleotides or ribonucleotides, preferably more than three. Its exact size will depend on many factors, which in turn depend on the ultimate function or use of the oligonucleotide. An oligonucleotide can be derived synthetically or by cloning. Chimeras of deoxyribonucleotides and ribonucleotides may also be within the scope of the present invention.

Sequence Amplification. A method for generating large amounts of a target sequence. In general, one or more amplification primers are annealed to a nucleic acid sequence. Using appropriate enzymes, sequences found adjacent to, or in between the primers are amplified.

Amplification Primer. An oligonucleotide which is capable of annealing adjacent to a target sequence and serving as an initiation point for DNA synthesis when placed under conditions in which synthesis of a primer extension product which is complementary to a nucleic acid strand is initiated.

Antisense nucleic acid molecule. An "antisense nucleic acid molecule" refers herein to a molecule capable of forming a stable duplex or triplex with a portion of its targeted nucleic acid sequence (DNA or RNA). The design and modification of antisense nucleic acid molecules is well known in the art as described for example in WO 96/32966, WO 96/11266, WO 94/15646, WO 93/08845, and USP 5,593,974. Antisense nucleic acid molecules, as sense oligos, can be derived from the nucleic acid sequences of the present invention and modified in accordance to well known methods. For example, some antisense molecules (or sense oligos or sequences) can be designed to be more resistant to degradation, or if required, to increase their affinity to their targeted sequence, to affect their transport to chosen cell types or cell compartments, and/or to enhance their lipid solubility by using nucleotide analogs and/or substituting chosen chemical fragments thereof, as commonly known in the art PCA3 gene has also been described as DD3^{PCA3}, the sequence of which is also found as GenBank's accession number AF103907.

### BRIEF DESCRIPTION OF THE DRAWINGS

Having thus generally described the invention, reference will now be made to the accompanying drawings, showing by way of illustration a preferred embodiment thereof, and in which:

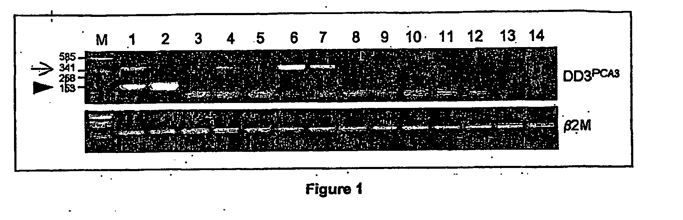

Figure 1: Expression of PCA3 in several human tissues using 32 cycles of PCA3-specific RT-PCR with the following primers: forward 5'-CAGGAAGCACAAAAGGAAGC-3'(SEQ ID NO:3) and reverse 5'-TCCTGCCCATCCTTTAAGG-3' (SEQ ID NO:4). The-following tissues have been analyzed: normal prostate (1), prostate cancer (2), testis (3), heart (4), lung (5), artery (6), kidney (7), liver (8), breast cancer (9), normal breast (10), cervix (11), endometrium (12), ovarium (13), and kidney cancer (14). The arrowhead indicates the spliced PCA3 transcript (151 bps) in prostate samples only and the arrow the non-spliced transcript (378 bps) in the other tissues. Note that the signals in lanes 1 and 2 are saturated. A beta-2-microglobulin PCR was performed as a control (lower panel)..
Figure 2: Schematic representation of the PCA3 transcription unit. Boxes indicate the four PCA3 exons; the solid arrowhead the prostate-specific PCA3 promoter and the arrows indicate the different (*putative*) PCA3 transcripts.
Figure 3: PCA3 expression by RT-PCR Expression of PCA3 was monitored in several human tissues using 32 cycles of PCA3-specific RT-PCR Different primers were used. The position thereof with respect to the PCA3 sequence described in GenBank as accession number AF103907 is indicated:
   Forward primers:
      BUS 1 (AGAAGCTGGCATCAGAAAAA, SEQ ID No: 12; exon 1, pos. 23-42); AH1 (CAGGAAGCACAAAAGGAAGC, SEQ ID NO:3; exon 3, pos. 443-462); BUS10 (ATCCCTGGGAGAAATGCC, SEQ ID NO:42; exon 4a, pos. 469-486); BUS17 (CACACAGCATGATCATTACGG, SEQ ID NO:43; exon 4b, pos. 1129-1149);
   Reverse primers:
      BUS7 (CTGGAAATGTGCAAAAACAT, SEQ ID NO:44; exon 3, pos. 420-401); AH2 (TCCTGCCCATCCTTTAAGG, SEQ ID NO:4; exon 4a, pos. 593-575); BUS11 (GTTGCATGTCTTGTGAAGCC, SEQ ID NO:45; exon 4a, pos. 719-700); BUS16 (TGATGGTGATGACAGATAAGGC, SEQ ID NO:46; exon 4b, pos. 1482-1461). The following tissues were analyzed: lane 1, H₂O; lane 2, seminal vesicle; lane 3, heart; lane 4, spleen; lane 5, lung; lane 6, bladder, lane 7, bladder-RT; lane 8, prostate cancer; lane 9, normal prostate; lane 10, prostate cancer, lane 11, normal prostate; and lane 12, LNCaP (cell line).

Other objects, advantages and features of the present invention will become more apparent upon reading of the following non-restrictive description of preferred embodiments with reference to the accompanying drawings which are exemplary and should not be interpreted as limiting the scope of the present invention.

### DESCRIPTION OF THE PREFERRED EMBODIMENT

### I. Synthesis of Nucleic Acid

Isolated nucleic acid molecules as described herein are meant to include those that result from any known method, such as chemically synthesized Similarly, an oligomer which corresponds to the nucleic acid molecule, or to each of the divided fragments, can be synthesized. Such synthetic oligonucleotides can be prepared, for example, by the triester method of Matteucci et al., J. Am. Chem Soc. 103:3185-3191 (1981) or by using an automated DNA synthesizer.

An oligonucleotide can be derived synthetically or by cloning. If necessary, the 5'-ends of the oligomers can be phosphorylated using T4 polynucleotide kinase. Kinasing of single strands prior to annealing or for labeling can be achieved using an excess of the enzyme. If kinasing is for the labeling of probe, the ATP can contain high specific activity radioisotopes. Then, the DNA oligomer can be subjected to annealing and ligation with T4 ligase or the like.

### II. A Nucleic Acid for the Specific Detection of PCA3 Nucleic Acid

The present invention relates to a nucleic acid for the specific detection, in a sample, of the presence of PCA3 nucleic acid sequences which are associated with prostate cancer, comprising the above-described nucleic acid molecules or at least a fragment thereof which binds under stringent conditions to PCA3 nucleic acid.

In one preferred embodiment, the present invention relates to oligomers which specifically target and enable amplification (i.e. primers) of PCA3 RNA sequences associated with prostate cancer.

The amplified product can be detected following hybridizing with a probe which consists of an isolated nucleic acid consisting of 10 to 1000 nucleotides (prefererably, 10 to 500, 10 to 100, 10 to 50, 10 to 35, 20 to 1000, 20 to 500, 20 to 100, 20 to 50, or 20 to 35) which hybridizes preferentially to an amplified product which originated from PCA3 RNA associated with prostate cancer, but preferentially not the PCA3 gene, wherein said nucleic acid probe is or is complementary to a nucleotide sequence consisting of at least 10 consecutive nucleotides (preferably, 15, 1-8, 20, 25, or 30) from the nucleic acid molecule comprising a polynucleotide sequence at least 90% identical to a sequence selected from the group consisting of:
(a) a region of the nucleotide sequence of PCA3 SEQ ID NO:1 or 2, which is associated with prostate cancer;
(b) a nucleotide sequence which spans two exon junctions, preferably exons 1 and 2, exons 1 and 3 (exon 1 being contiguous to exon 3, through alternative splicing), and exons 3 and 4;
(c) a nucleotide sequence which spans a sufficient number of the PCA3 exon junctions, wherein the exon junctions are defined as follows: exon junction of exons 1 and 2, nucleotide positions 98-99 as set forth in SEQ ID NO:1; exon junction of exons 2 and 3, nucleotide positions 263-264 as set forth in SEQ ID NO:1; exon junction of exons 3 and 4a, nucleotide positions 446-447 as set forth in SEQ ID NO:1; and exon junction of exons 4a and 4b, nucleotide positions 985-986 as set forth in SEQ ID NO:1;
(d) a nucleotide sequence which spans a sufficient number of the PCA3 exon junctions, wherein the exon junctions are defined as follows: exon junction of exons 1 and 2, nucleotide positions 120-121 as set forth in SEQ ID NO:2; exon junction of exons 2 and 3, nucleotide positions 285-286 as set forth in SEQ ID NO:2; exon junction of exons 3 and 4a, nucleotide positions 468-469 as set forth in SEQ ID NO:2; exon junction of exons 4a and 4b, nucleotide positions 1007-1008 as set forth in SEQ ID NO:2; exon junction of exons 4b and 4c, nucleotide positions 2066-2067 as set forth in SEQ ID NO:2; and exon junction of exons 4c and 4d, nucleotide positions 2622-2623 as set forth in SEQ ID NO:2.

Preferably, a probe in accordance with the present invention does not specifically hybridize to nucleotides 511-985 of SEQ ID NO:1, to nucleotides 567-961 of SEQ ID NO:1, to nucleotides 533-1007 of SEQ ID NO:2, or to nucleotides 589-983 of SEQ ID NO:2.

Complementary sequences are also known as antisense nucleic acids when they comprise sequences which are complementary to the coding strand. Herein, SEQ ID NOs: 1 and 2 are arbitrarily defined as being the coding strands.

Primers in accordance with the present invention can be designed as commonly known in the art based on the sequences of PCA3 provided herein. More preferably, the primers will be chosen to amplify a PCA3 RNA which is associated with prostate cancer. One such PCA3 RNA is a PCA3 RNA which lacks intron 1 (between exons 1 and 2). Another prostate-cancer specific PCA3 RNA in accordance with the present invention, lacks the intron between exon 3 and exon 4a. Of course different permutations of such prostate-cancer specific PCA3 RNAs are also encompassed by the present invention. For example, three non-limiting prostate-cancer specific PCA3 RNAs include a PCA3 RNA lacking at least intron 1, and PCA3 RNAs having the following contiguous exons: exons 1, 2, 3, 4a, 4b, 4c and 4d; and exons 1, 3, 4a, 4b, 4c and 4d.

In a preferred embodiment of the present invention, a primer which is designed to bind to exon 1 is used, together with a second primer designed to bind to exon 3 or to exon 4. Since intro 1 is a large intron (approximately 20 kb), the amplifying conditions can be selected so as to inhibit the production of such a large amplification product, should the intron be present in the PCA3 sequence. Alternatively, the conditions of amplification can be selected so as to enable the amplification of such large products. In such an embodiment, the presence of intron 1 in the PCA3 RNA can be ascertained by numerous means known in the art (including using an intronic probe and/or a probe which designed to bind to contiguous exon 1 exon 2 sequences; two non-limiting examples thereof is shown in Table 1). It will be recognized by the person of ordinary skill that the position of the primer at the exon junction and the length of the primer can be varied, as known in the art

In another preferred embodiment, a primer which is designed to bind to exon 1 is used, together with a second primer designed to bind an exon junction region of the present invention. Since exon 1 has been shown to be one preferred targeted exon to amplify prostate-cancer specific RNAs, such an embodiment is especially preferred since it can generate prostate cancer specific amplification products. However; targeting another exon of PCA3 is also another preferred embodiment. For example, exon 3 and exon 4 can be targeted (see below).

Examples of nucleic acid primers which can be derived from the exon sequences shown hereinbelow and specific primers designed to amplify an exon junction of the present invention are set forth in Table 1, below.

**Table 1: Nucleic Acid Printers**

| **Nucleic Acid Region** | **Size** | **Nucleotides** | **Size** | **Nucleotides** |
|---|---|---|---|---|
| *Exon Sequence from Which to Derive Primers* | | | | |
| Exon 1 | 98 | 1-98 of SEQ ID NO:1 | 120 | 1-120 of SEQ ID NO:2 |
| Exon 2 | 165 | 99-263 of SEQ ID NO:1 | 165 | 121-285 of SEQ ID NO:2 |
| Exon 3 | 183 | 264-446 of SEQ ID NO:1 | 183 | 286-468 of SEQ ID NO:2 |
| Exon 4a | 539 | 447-985 of SEQ ID NO:1 | 539 | 469-1007 of SEQ ID NO:2 |
| Exon 4b | 1052 | 986-2037 of SEQ ID NO:1 | 1059 | 1008-2066 of SEQ ID NO:2 |
| Exon 4c | - | - | 56 | 2067-2622 of SEQ ID NO:2 |
| Exon 4d | - | - | 960 | 2623-3582 of SEQ ID NO:2 |

| *Exon Junction Specific Primers* | | | | |
|---|---|---|---|---|
| Exon Junction 1 | 20 | 89-108 of SEQ ID NO:1 (SEQ ID NO:5) | 20 | 109-128 of SEQ ID NO:2 (SEQ ID NO:6) |
| Exon Junction 2 | 20 | 252-271 of SEQ ID NO:1 (SEQ ID NO:7) | 20 | 274-293 of SEQ ID NO:2 (SEQ ID NO:7) |
| Exon Junction 3 | 20 | 435-454 of SEQ ID NO:1 (SEQ ID NO:8) | 20 | 457-476 of SEQ ID NO:2 (SEQ ID NO:8) |
| Exon Junction 4 | 20 | 974-993 of SEQ ID NO:1 (SEQ ID NO:9) | 20 | 996-1015 of SEQ ID NO:2 (SEQ ID NO:9) |
| Exon Junction 5 | - | - | 20 | 2055-2074 of SEQ ID NO:2 (SEQ ID NO:10) |
| Exon Junction 6 | - | - | 20 | 2611-2630 of SEQ ID NO:2 (SEQ ID NO:11) |

While the present invention can be carried out without the use of a probe which targets RCA3 sequences, and preferably the exon junctions of PCA3 in accordance with the present invention, such probes can add a further specificity to the methods and kits of the present invention. Non limiting examples of specific nucleic acid probes which can be used in the present invention (and designed based on the exonic sequences shown in Table 1) are set forth in Table 2, below.

**Table 2: Nucleic Acid Probes**

| **Size** | **Nucleotides** | **Sequence** | **SEQ ID NO:** |
|---|---|---|---|
| 20 | 1-20 of SEQ ID NO:1 | AGAAGCTGGCATCAGAAAAA | 12 |
| 30 | 1-30 of SEQ ID NO:1 | AGAAGCTGGCATCAGAAAAACAGAGGGGAG | 13 |
| 40 | 1-40 of SEQ ID NO:1 | AGAAGCTGGCATCAGAAAAACAGAGGGGAGATTTGTGTGG | 14 |
| 20 | 89-108 of SEQ ID NO:1 | TGATACAGAGGAATTACAAC | 5 |
| 30 | 257-286 of SEQ ID NO:1 | GGCAGGGGTGAGAAATAAGAAAGGCTGCTG | 15 |
| 20 | 274-293 of SEQ ID NO:1 | AGAAAGGCTGCTGACTTTAC | 16 |
| 20 | 1-20 of SEQ ID NO:2 | ACAGAAGAAATAGCAAGTGC | 17 |
| 30 | 1-30 of SEQ ID NO:2 | ACAGAAGAAATAGCAAGTGCCGAGAAGCTG | 18 |
| 40 | 1-40 of SEQ ID NO:2 | ACAGAAGAAATAGCAAGTGCCGAGAAGCTGGCATCAGAAA | 19 |
| 30 | 114-143 of SEQ ID NO:2 | TACAGAGGAATTACAACACATATACTTAGT | 20 |
| 20 | 284-303 of SEQ ID NO:2 | GGGTGAGAAATAAGAAAGGC | 21 |

Of course, as will be understood by the person of ordinary skill, a multitude of additional probes can be designed from the same or other region of SEQ ID NO:1 as well as from SEQ ID NO:2 and other sequences of the present invention, whether they target exon junctions or not

The hybridization probes of the present invention can be labeled by standard labeling techniques such as with a radiolabel, enzyme label, fluorescent label, biotin-avidin label, chemiluminescence, and the life. After hybridization, the probes can be visualized using known methods.

The nucleic acid probes of the present invention include RNA, as well as DNA probes, such probes being generated using techniques known in the art.

In one embodiment of the above described method, a nucleic acid probe is immobilized on a solid support. Examples of such solid supports include, but are not limited to, plastics such as polycarbonate, complex carbohydrates such as agarose and sepharose, and acrylic resins, such as polyacrylamide and latex beads. Techniques for coupling nucleic acid probes to such solid supports are well known in the art.

The test samples suitable for nucleic acid probing methods described herein include, for example, cells or nucleic acid extracts of cells, or biological fluids. The sample used in the above-described methods will vary based on the assay format, the detection method and the nature of the tissues, cells or extracts to be assayed. Methods for preparing nucleic acid extracts of cells are well known in the art and can be readily adapted in order to obtain a sample which is compatible with the method utilized. In the present invention, the sample is a urine sample.

### III. A Method of Detecting the Presence of PCA3 Nucleic Acid in a Sample

In another embodiment, the present invention relates to a method of detecting the presence of prostate cancer specific PCA3 nucleic acid in a sample comprising a) contacting the sample with the above-described nucleic acid primers, under specific amplification conditions, and b) detecting the presence of the amplified product. One skilled in the art would select the nucleic acid primers according to techniques known in the art as described above. In one particular embodiment one of the primers binds to exon 1 of PCA3. In another embodiment,-exon-3 or exon 4 of PCA3 is targeted by the primer. In another embodiment a probe is used to identify the amplification product. Samples to be tested include but should not be limited to RNA samples from human tissue.

### IV. A Kit for Detecting the Presence of PC43 Nucleic Acid in a Sample

In another embodiment, the present invention relates to a kit for detecting the presence of prostate cancer specific PCA3 nucleic acid in a sample comprising at least one container means having disposed therein at least one primer pair, (e.g. one binding to exon 1, the other to exon 3; one binding to exons 1, the other to exon 4a; and one binding to exon 1, the other to oxon3 - exon4a junction). In a preferred embodiment, the kit further comprises other containers comprising one or more of the following: amplification reagents, probes, wash reagents and reagents capable of detecting the presence of bound nucleic acid probe. Examples of detection reagents include, but are not limited to radiolabelled probes, enzymatic labeled probes (horse radish peroxidase, alkaline phosphatase), and affinity labeled probes (biotin, avidin, or steptavidin).

In detail, a compartmentalized kit includes any kit in which reagents are contained in separate containers. Such containers include small glass containers, plastic containers or strips of plastic or paper. Such containers allow the efficient transfer of reagents from one compartment to another compartment such that the samples and reagents are not cross-contaminated and the agents or solutions of each container can be added in a quantitative fashion from one compartment to another. Such containers will include a container which will accept the test sample, a container which contains the probe or primers used in the assay, containers which contain wash reagents (such as phosphate buffered saline, Tris-buffers, and the like), and containers which contain the reagents used to detect the hybridized probe, bound antibody, amplified product, or the like.

One skilled in the art will readily recognize that the nucleic acid probes described in the present invention can readily be incorporated into one of the established kit formats which are well known in the art.

### V. Diagnostic Screening

It is to be understood that although the following discussion is specifically directed to human patients, the teachings are also applicable to any animal that expresses PCA3.

The diagnostic and screening methods of the invention are especially useful for a patient suspected of being at risk for developing a disease associated with an altered expression level of PCA3 based on family history, or a patient in which it is desired to diagnose a PCA3-related disease (ex. prostate cancer).

Presymptomatic screening of an individual in need of such screening is now possible using DNA encoring the PCA3 protein or the PCA3 gene or fragments thereof as described herein. The screening method allows a presymptomatic diagnosis, including prenatal diagnosis, of the presence of a missing or aberrant PCA3 gene in individuals, and thus an opinion concerning the likelihood that such individual would develop or has developed a PCA3-associated disease. This is especially valuable for the identification of carriers of altered or missing PCA3 genes, for example, from individuals with a family history of a PCA3-associated disease (e.g. prostate cancer, urogenital cancer). Early diagnosis is also desired to maximize appropriate timely intervention.

In one preferred embodiments of the method of screening, a tissue sample would be taken from such individual; and screened for the presence of prostate cancer-specific PCA3 nucleic acid.

More specifically, a method of diagnosing the presence or predisposition to develop prostate cancer in a patient is provided herein.

The screening and diagnostic methods of the invention do not require that the entire PCA3 sequence be used for the probe. Rather, it is only necessary to use a fragment or length of nucleic acid that is sufficient to detect the presence of the PCA3 nucleic acid from a normal or affected individual, the absence of such nucleic acid, or an altered structure of such nucleic acid (such as an aberrant splicing pattern). Preferably, any of the probes as described herein are used.

Non-limiting embodiments of the present invention also include an assay for detecting PCA3 RNA in which sequences in the spliced RNA are amplified and detected by using a probe that specifically hybridizes to a chosen exon-exon junction of PCA3 that is in the amplified nucleic acid. Generally, one primer in the amplification reaction hybridizes specifically to a sequence in a first exon (or upstream exon) and the other primer used in the amplification reaction hybridizes specifically to a sequence in a second exon (or downstream exon), and the probe hybridizes to a sequence that spans the 3' region of the first exon and the 5' region of the second-exon. That is, the probe is specific for a chosen exon-exon junction in an amplified sequence made from a spliced PCA3 RNA that lacks at least one intron between the upstream and downstream exon sequences to which the primers hybridize. Primers for use in amplifying sequences of the spliced RNA that contain a chosen exon-exon junction can readily be determined by using standard methods, so long as the region amplified by the primer pair contains the exon-exon junction sequence or its complementary sequence. Any method of nucleic acid amplification may be used to amplify the sequence that contains the chosen exon-exon junction and procedures for using any of a variety of well-known amplification methods can readily be determined by those skilled in the art.

Probes that detect a chosen exon-exon junction may be labeled with any of a variety of labels that can, directly or indirectly, result in a signal when the probe is hybridized to the amplified sequence that contains the exon-exon junction. For example, a label may be any moiety that produces a luminescent, fluorescent, radioactive, or enzymatic signal that can be detected by using methods well known in the art A probe need not be labeled with a label moiety if binding of the probe specifically to the amplified nucleic acid containing the exon-exon junction results in a detectable signal, such as, for example a delectable electrical impulse. Non-limiting examples of amplification primers for exon sequences flanking a chosen exon-exon junction and probes specific for some PCA3 junctions are shown herein and exemplified hereinbelow (e.g. Table 3), and are representative of non-limiting embodiments of an assay to detect spliced PCA3 RNA in a sample. Additional exemplary details of procedures that may be used in such assays are described in Examples 4 and 5.

The present invention is described in further detail in the following non-limiting examples.

### EXAMPLE 1

### PCA3 is a specific marker for prostate cancer

Gandini *et al.,* 2003 claim that the prostate-specific expression of PCA3 is restricted to exons 4 of the PCA3 gene. The authors show that RT-PCR amplification of the PCA3 transcript using primers specific for exons 1 and 3 also amplified a PCA3-specific product in several non-prostate, tissues and cell lines.
After the first description of the PCA3 gene, the exon 1 forward and exon 3 reverse PCR primers were used exactly as described in the letter by Gandini *et al. supra.* In the past four years, PCA3 was amplified in many samples using these primers, and non-prostatic expression of PCA3 has yet to be observed Although it is not clear from the letter how many cycles of PCR amplification Gandini *et al. supra* performed, more than 35 rounds of amplification were never used for the results described in this example. It cannot be excluded that using more rounds of amplification might result in detection of low levels of expression. These levels of expression, however, would be far below those observed in prostate cancer, normal prostate and even prostate cancer cell lines.

One interesting observation made herein is that PCA3 can be amplified in non-prostatic tissues, using PCA3-specific primers spanning exons 3 and 4 (Fig.1). The level of expression is lower than in normal prostatic tissue and far below the expression in prostate cancer tissue. Strikingly, the PCA3 transcripts in non-prostatic tissues are not spliced like they are in prostate-derived samples. In normal prostatic tissue the non-spliced transcript is expressed at low levels. In prostate tumor tissue the non spliced variant is not expressed or not detectable due to the high overexpression of spliced PCA3 that may be preferentially amplified in the PCR reactions. In RNA samples not subjected to reverse transcription, no amplification product was found, indicating that the non-spliced PCA3 PCR products were not attributable to DNA contamination.

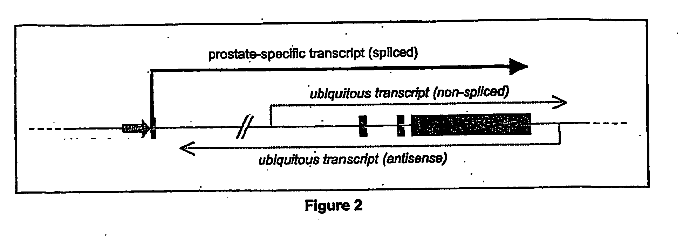

Several explanations for the presence of non-spliced PCA3 transcripts can be postulated (Fig.2). Firstly, in prostatic tissues the PCA3 transcript may be tissue-specifically spliced, a phenomenon that has been described for several other genes (Black, 2003). Secondly, an alternative ubiquitous promoter may exist in the PCA3 gene, resulting in a second transcript that is not prostate-specific. This option seems less likely, since the transcript is not spliced despite the strong splice consensus sequences flanking the PCA3 exons (Bussemakers *et al.* supra). Thirdly, a ubiquitous promoter may be present at the 3' end of the PCA3 gene in reverse orientation, leading to an antisense PCA3 transcript in most tissues: It has recently been reported that antisense transcription occurs widespread in the human genome (Yelin *et al*., 2003), and therefore it is not unlikely that an antisense PCA3 transcript exists. Such antisense transcripts are often involved in gene regulation processes (Yelin *et al*.,supra). Therefore, such a putative PCA3 antisense transcript may be involved in the regulation of the PCA3 transcription in prostate cells, or *vice versa* in prostate cells the PCA3 transcript may affect the, so far unidentified, antisense transcribed gene. Currently, whether alternative splicing or alternative transcription initiation mechanisms are responsible for the observed non-prostatic PCA3-like transcript, is being investigated.

### EXAMPLE 2

### PCA3 expression by RT-PCR

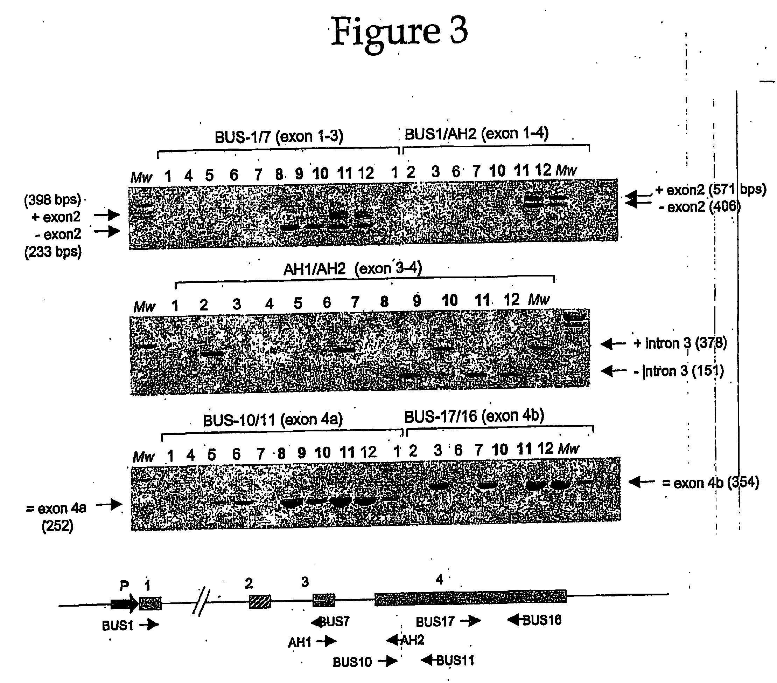

With respect to Figure 3, transcription of the PCA3 gene or a PCA3-like gene is evident in tissues other than the prostate. However, these transcripts are either not spliced or are complementary (*i.e*. antisense) to the PCA3 gene. To date the observation of any alternatively spliced PCA3 variant (*e.g*. exon 1 to 3 product) in non-prostatic tissues has not occurred. For the application of PCA3 as a marker for prostate cancer this has one major implication: preferred primers for the amplification of the PCA3 transcripts in patient samples should, in one embodiment, cross the large (16kb) first intron. This region of the PCA3 gene may be present in the alternative non-spliced or antisense transcripts, but is lacking from the prostate-specific spliced form of PCA3. Therefore, using exon 1 to exon. 3 or 4 primer pairs, is one preferred means according to the present invention to detect amplified prostate-specific spliced form of PCA3 (especially in conditions whereby the large intron prevents amplification of this region in the non-spliced transcripts). Two independent assays for the detection of PCA3 RNA in patient material have been developed, using an exon 1 forward and an exon 4 reverse primer and exon 4-specific detection probes (De Kok *et al.,* 2002; Hessels *et al.,* 2003). The PCA3 detection assays have been applied on over 200 patient samples and have been shown to be very specific and sensitive with a strong negative predictive value (Hessels supra). Analysis of over 100 control samples has yet to result in non-specific amplification products.

### EXAMPLE 3

### Assay to Detect Spliced PCA3 RNA Using Exon-Exon Junction Probes

This example illustrates some non-limiting embodiments of the assay for detecting PCA3 RNA in which sequences in the spliced RNA are amplified and-detected by using a probe that specifically hybridizes to a chosen exon-exon junction of PCA3 that is in the amplified nucleic acid. Generally, one primer in the amplification reaction hybridizes specifically to a sequence in a first exon (or upstream exon) and the other primer used in the amplification reaction hybridizes specifically to a sequence in a second exon (or downstream exon), and the probe hybridizes to a sequence that spans the 3' region of the first exon and the 5' region of the second exon. That is, the probe is specific for a chosen exon exon junction in an amplified sequence made from a spliced PCA3 RNA that lacks at least one intron between the upstream and downstream exon sequences to which the primers hybridize. Primers for use in amplifying sequences of the spliced RNA that contain a chosen exon-exon junction can readily be determined by using standard methods so long as the region amplified by the primer pair contains the exon-exon junction sequence or its complementary sequence. Any method of nucleic acid amplification may be used to amplify the sequence that contains the chosen exon-exon junction and procedures for using any of a variety of well-known amplification methods can readily be determined by those skilled in the art.

Probes that detect a chosen exon-exon junction may be labeled with any of a variety of labels that can, directly or indirectly, result in a signal when the probe is hybridized to the amplified sequence that contains the exon-exon junction. For example, a label may be any moiety that produces a colorimetric, luminescent, fluorescent, radioactive, or enzymatic signal that can be detected by using methods well known in the art. A probe need not be labeled with a label moiety if binding of the probe specifically to the amplified nucleic acid containing the exon-exon junction results in a detectable signal, such as, for example a detectable electrical impulse.

Examples of amplification primer pair combinations that amplify nucleic acid sequence that includes an exon-exon junction and embodiments of some exon-exon junction probe sequences are shown in Table 3. It will be understood by those skilled in the art that the probe sequences shown below also include the complementary sequences of the sequences shown, and sequences that include insignificant changes to the specific sequences shown (i.e., the changes do not affect the ability of a probe to hybridize specifically to the chosen exon-exon junction sequence, under standard hybridization conditions). Furthermore, although the probe sequences are shown as DNA sequences, those skilled in the art will understand that the corresponding RNA sequences or their complementary sequences may be used as probes. Also, the backbone linkages of the probe base sequences may include one or more standard RNA linkages, DNA linkages, mixed RNA-DNA linkages, or other linkages such as 2'-*O*-methyl linkages or peptide nucleic acid linkages, all of which are well known to those skilled in the art.

As shown in Table 3 (first column), the chosen exon-exon junction to be detected may join exons 1 and 2 (exon 1/exon 2), exons 1 and 3 (exon 1/exon 3), exons 2 and 3 (exon 2/exon 3), or exons 3 and 4 (exon 3/exon 4). Primer pairs are sequences located in two different exons that directly or indirectly flank the chosen exon-exon junction (Table 3, second column). Thus, for an exon 1/exon 2 junction, the primer pairs are one primer specific for a sequence contained in exon 1 and another primer specific for a sequence contained in exon 2. But for detecting an exon 2/exon 3 junction or an exon 3/exon 4 junction, the primer pairs may be selected from more than two different exons (see below in column 2) so long as the amplified sequence contains the chosen exon-exon junction region. The "exon 4" primers include primers specific for a sequence contained in any sequence of exons 4a, 4b, 4c, or 4d.

**Table 3**

| **Exon Junction Detected** | **Primer Pairs in PCA3 Exons** | **Exon Junction Probes** | **SEQ ID NO:** |
|---|---|---|---|
| Exon 1/ exon 2 | exon 1 and exon 2 | GGACCTGATGATACAGAGGAATTAC | 22 |
| Exon 1/ exon 2 | exon 1 and exon 2 | GAGGAATTACAACAC | 23 |
| Exon 1/ exon 2 | exon 1 and exon 2 | GATGATACAGAGGAATTACAACAC | 24 |
| Exon 1/ exon 3 | exon 1 and exon 3 | GATGATACAGAGGTGAGAAATAAG | 25 |
| Exon 1/ exon 3 | exon 1 and exon 3 | CAGAGGTGAGAAATAAGAAAGGC | 26 |
| Exon 1/ exon 3 | exon 1 and exon 3 | GATACAGAGGTGAGAAATAAG | 27 |
| Exon 1/ exon 3 | exon 1 and exon 3 | GATACAGAGGTGAGAAATAAGAAAGGCTGCTGAC | 28 |
| Bxon 2 /exon 3 | exon 2 and exon 3, orexon 1 and exon 3 | GGCAGGGGTGAGAAATAAG | 29 |
| Exon 2 / exon 3 | exon 2 and exon 3, or | CTCAATGGCAGGGGTGAG | 30 |
| | exon 1 and exon 3 | | |
| Exon 2 / exon 3 | exon 2 and exon 3, or | CTCAATGGCAGGGGTGAGAAATAAGAAAGGCTGCTGAC | 31 |
| | exon 1 and exon 3 | | |
| Exon 3 / exon 4 | exon 3 and exon 4, or exon 1 and | GGAAGCACAGAGATCCCTGG | 8 |
| | exon 4, or | | |
| | exon 2 and exon 4 | | |
| exon 3 / exon 4 | exon 3 and exon 4, or | GCACAAAAGGAAGCACAGAGATCCCTGGGAG | 32 |
| | exon 1 and exon 4, or | | |
| | exon 2 and exon 4 | | |
| exon 3 / exon 4 | exon 3 and exon 4, or | GCACAGAGATCCCTGGGAG | 33 |
| | exon 1 and exon 4, or | | |
| | exon 2 and exon 4 | | |
| exon 3 / exon 4 | exon 3 and exon 4, or | GCACAGAGGACCCTTCGTG | 34 |
| | exon 1 and exon 4, or | | |
| | exon 2 and exon 4 | | |
| exon 3 / exon 4 | exon 3 and exon 4, or | GGAAGCACAAAAGGAAGCACAGAGATCCCTGGG | 35 |
| | exon 1 and exon 4, or | | |
| | exon 2 and exon 4 | | |

These non-limiting examples of amplification primers for exon sequences flanking a chosen exon-exon junction and probes specific for some PCA3 junctions are representative of embodiments of the assay to detect spliced PCA3 RNA in a sample.

Additional details of procedures and embodiments that may be used in such assays are described in the examples that follow. Those skilled in the art will appreciate that variations of these procedures and reagents may be used that would not materially affect the results or conclusions drawn from these experiments and, therefore, these examples describe non limiting embodiments of the invention.

### EXAMPLE 4

### Sensitivity of the PCA3 Amplification Assay

This example demonstrates the sensitivity of an amplification assay targeting the exon3-exon4 spliced variant of the PCA3 mRNA. For use in these experiments, oligonucleotides were synthesized using standard phosphoramidite chemistry (e.g., as described in Caruthers et al., Methods in Enzymol., 154:287 (1987)), performed by using an automated system (Expedite^{™} 8909 Nucleic Acid Synthesizer, Applied Biosystems, Foster City, CA).

Nucleic acid primers were designed for use in transcription mediated amplification (fMA), a procedure disclosed in detail previously (e.g., Kacian et al., U.S. Patent Nos. 5,399,491 and 5,480,784). TMA is an isothermal amplification procedure that produces more than a billion-fold increase in copy number of the target sequence by using reverse transcriptase and RNA polymerase. Briefly, in TMA a single-stranded target sequence is used to synthesize a double-stranded DNA intermediate by using reverse transcriptase in the presence of a pair of amplification oligonucleotides, one of which has a 5' RNA polymerase promoter sequence. The DNA intermediate includes a double-stranded promoter sequence that is recognized by a RNA polymerase and directs transcription of the target sequence (i.e., hundreds of copies of RNA). Each RNA transcript is then converted to a double-stranded DNA intermediate which is used to produce additional RNA and, thus, the reaction proceeds exponentially..

The primers were synthesized with the following sequences: CAGGAAGCACAAAAGGAAGC (SEQ ID NO:3) and AATTTAATACGACTCACTATAGGGAGAGGCTCATCGATGACCCAAGAT GG (SEQ ID NO:36). The underlined 5' portion of the second primer (called a "promoter primer") is a T7 promoter sequence (AATTTAATACGACTCACTATAGGGAGA, SEQ ID.NO:37) which is used in the TMA procedure, but those skilled in the art will appreciate that a primer made up of the 3' target-specific sequence (GGCTCATCGATGACCCAAGATGG, SEQ ID NO:38) or its complementary sequence could equivalently be used in an amplification reaction that does not involve T7 RNA polymerase transcription. These primers were designed to amplify across the splice junction between exons 3 and 4 of the PCA3 mRNA. A probe (GGAAGCACAGAGATCCCTGG, SEQ ID NO:8) was used which spans the exon3-exon4 splice junction. That is, the probe was designed to specifically detect only amplicon derived from that spliced mRNA, and not the exon3-intron3-exon4 unspliced form. The probe was synthesized *in vitro* to include a non-nucleotide linker (see Arnold et al., U.S. Patent Nos. 5,585,481 and 5,639,604), and labeled with a chemiluminescent acridinium ester (see Arnold et al., U.S. Pat. No. 5,185,439). The RNA target for amplification was an *in vitro* transcript that contained the sequence of the exonl-exon3-exon4 spliced form of the PCA3 mRNA. Skilled artisans will appreciate that the target could be produced by other standard methods, such as, e.g., chemical lysis of cells by using a detergent-containing buffered solution that inhibits RNAse activity, including targets purification (e.g., as described by Weisburg et al., in US Patent No. 6,110,678).

Three sample tubes were used for each of the target levels tested (n=3), in the range of 0 to 10,000 copies of target per reaction (see Table 4).To each reaction tube, Amplification Reagent (75 µL of a solution containing 26.7 mM rATP, 5.0 mM rCTP, 33.3 mM rGTP and 5.0 mM rUTP, 125 mM HEPES, 8% (w/v) trehalose dihydrate, 1.33 mM dATP, 1.33 mM dCTP, 1.33 mM dGTP, 1.33 mM dTTP, 33 mM KCl, 30.6 mM MgCl₂, 0.10% (w/v) methyl paraben, 0.02% (w/v) propyl paraben, and 0.003% phenol red, at pH 7.5) containing these primers (15 pmol each) was added. Target RNA transcript was then added to the tubes in 10 µL of water, and mixed, and then each reaction tubes received 200 µL of silicone oil (United Chemical Technologies, Inc., Bristol, PA), was covered and vortexed for about 10 seconds before being incubated in a 62°C water bath for about 10 minutes for an initial annealing step in which binding of the promoter-primer to the target nucleic acid occurred. Reaction tubes were then incubated at 42°C for about 5 minutes, and then 25 µL of the Enzyme Reagent (50 mM HEPES, 125 mM N-acetyl-L-cysteine, 120 mM KCl, 1 mM EDTA, 20% (vlv) glycerol, 10.2% (v/v) TRITON X-100, 0.2 M trehalose, 0.90 U/mL Moloney murine leukemia virus (MMLV) reverse transcriptase (RT), and 0.20 U/mL T7 RNA polymerase, at pH 7.0, wherein 1 U of RT activity is defined as synthesis and release of 5.75 fmol cDNA in 15 min at 37°C for MMLV RT, and 1 U of T7 RNA polymerase activity is defined as production of 5.0 fmol RNA transcript in 20 min at 37°C) was added to each reaction tube, mixed gently and incubated at 42°C for about 60 minutes.

For detection of PCA3 amplification products, the reaction tubes were removed to room temperature and 100 µL of the hybridization reagent (100 mM succinate, 2% (w/v) lithium lauryl sulfate, 100 mM lithium hydroxide, 15 mM aldrithiol-2, 1.2 M LiCl, 20 mM EDTA, 3.0% (v/v) ethanol, at pH 4.7) containing 100 fmol of a labeled detection probe and 400 pmol of unlabeled probe was added to each reaction tube. The labeled detection probe had an acridinium ester label joined to the probe by a non-nucleotide linker positioned between nucleotides 10 and 11, and the unlabeled probe was an oligomer of the same nucleic acid sequence but was not labeled. The reaction tubes were covered and vortexed for about 10 seconds and then incubated at 62°C for about 20 minutes to allow hybridization of the detection probe to amplification products. Reaction tubes were cooled at room temperature for about 5 minutes and then 250 µL of the Selection Reagent (600 mM boric acid, 182.5 mM NaOH, 1% (v/v) TRITON X-100, at pH 8.5) was added to each tube. Reaction tubes were covered, vortexed for about 10 seconds, and then incubated at 62°C for about 10 minutes to hydrolyze acridinium ester labels associated with unhybridized labeled probe. Reaction tubes were cooled to about 18° to 28°C for about 15 minutes and the chemiluminescent signal (in relative light units, or RLU) was detected by using a luminometer (LEADER7 450h or LEADER7 HC+ Luminometer, Gen-Probe Inc., San Diego, CA) equipped for automatic injection of Detection Reagent 1 (1 mM nitric acid, 32 mM hydrogen peroxide), followed by automatic injection of Detection Reagent 2 (1.5 M NaOH to adjust the pH to approximately neutral). The cut-off level for a negative result in this experiment was 50,000 RLU, i.e., positive samples provided a signal greater than 50,000 RLU.

The results shown in Table 4 show that the PCA3 assay detects a minimum-of-10 copies of the PCA3 RNA transcript The signal (RLU) obtained using 10 copies of the target was about 15-fold greater than the signal obtained for a negative sample (0 copies). The RLU signals detected correlated positively with the relative amount of target present in the samples, indicating the quantitative nature of this PCA3 assay system.

**Table 4: Sensitivity of the PCA3 Amplification Assay**

| **Target level (copies/reaction)** | **Average RLU (n=3)** |
|---|---|
| 0 | 1,306 |
| 10 | 19,989 |
| 100 | 1,012,272 |
| 1,000 | 2,749,382 |
| 10,000 | 3,726,173 |

### - EXAMPLE 5

### Amplification of PCA3 mRNA in cell pellets from male and female urine

This example demonstrates detection of the PCA3 exon3-exon4 spliced variant mRNA in cell pellets from urine. Unless otherwise specified, the reagents used were as described in Example 4. Cell pellets were prepared by centrifugation (1500 RCF for 15 min) of 200 µL of urine from each of three normal males and one normal female. Five replicates were prepared and analyzed from each subject. The urine supernatant was decanted, and the tubes drained. Each cell pellet was lysed in 400µL of Lysis Buffer (15 mM sodium phosphate monobasic, 15 mM sodium phosphate dibasic, 1.0 mM EDTA, 1.0 mM EGTA, 110 mM lithium lauryl sulfate, at pH 6.7, incubated 10 min at 62°C) to release target nucleic acids, and then cooled at room temperature for about 5 minutes.

To separate PCA3 mRNA target nucleic acid from other components present in the sample tubes, the contents of the sample tubes were transferred to clean tubes and combined with 100 µL of a Target Capture Reagent (250 mM HEPES, 310 mM LiOH, 1.88 M LiCl, 100 mM EDTA, at pH 6.4, and 250 µg/ml 1 micron magnetic particles (Sera-Mag^{™} MG-CM Carboxylate Modified, Seradyn, Inc., Indianapolis, Indiana) having (dT)₁₄ oligomers covalently bound thereto) containing 1.5 pmol of a target capture probe (AUCUGUUUUCCUGCCCAUCCUUUAAGTTT(A)₃₀, SEQ ID NO:39). This capture probe includes a 5' target binding region (AUCUGUUUUCCUGCCCAUCCUUUAAG, SEQ ID NO:40) and a 3' immobilized probe binding region (TTT(A)₃₀, SEQ ID NO:41). The tubes were covered, hand-shaken, incubated at 62°C for about 30 minutes to permit hybridization of the target binding region of the capture probe to the target nucleic acid, and cooled at room temperature for about 30 minutes for hybridization of the (dA)₃₀ sequence of the immobilized probe binding region of a complementary capture probe of (dT)₁₄ bound to magnetic particles. Then a magnetic field was applied for about 5 minutes to the outside of the tubes to isolate the magnetic particles with the bound nucleic acids, after which the sample solutions were aspirated from the tubes, and then the particles with bound nucleic acids in each tube were washed with a 1 mL of a Wash Solution (150 mM NaCl, 10 mM HEPES, 6.5 mM NaOH, 1 mM EDTA, 0.3% (v/v) ethanol, 0.1% (w/v) SDS, 0.02% (w/v) methylparaben, 0.01% (w/v) propylparaben, at pH 7.5, by vortexing for 10-20 sec), and again separated using a magnetic field (about 5 minutes) before the Wash Solution was aspirated away from the particles.

Following the target capture step, 75 µL of the Amplification Reagent containing primers described in Example 4 was added to each of the reaction tubes and then amplification, probe hybridization and detection were carried out as described in Example 4. The results shown in Table 5 indicate that the PCA3 assay amplified and detected PCA3-derived nucleic acid from male urine cell pellets, but not from female urine cell pellets. The range of RLU values obtained from the three normal males varied, indicating a difference in recovery of cells expressing PCA3, or a difference in PC3 mRNA expression. The RLU value obtained from the female urine cell pellet was at background, indicating that no PCA3 mRNA was detectable. The term "CV" in Table 5 stands for coefficient of variation and represents the standard deviation of the replicates over the mean of the replicates as a percentage.

**Table 5: Amplification of PCA3 mRNA in cell pellets from male and female urine**

| **Sample** | **Average RLU (n = 5)** | **%CV** |
|---|---|---|
| Male urine pellet 1 | 610,291 | 78.12% |
| Male urine pellet 2 | 1,255,240 | 16.75% |
| Male urine pellet 3 | 2,165,684 | 12.01% |
| Female urine pellet 1 | 1,063 | 0.93% |

### REFERENCES

1. Gandini, O., Luci, L., Stigliano, A., Lucera, R, Di Silverio, F., Toscano, V., and Cardillo, M.R. Is DD3 a new prostate-specific gene? Anticancer Res., 23 (1A):305-308 2003.
2. Bussemakers, M.J., van Bokhoven, A., Verhaegh, G.W., Smit, F.P., Karthaus, H.F., Schalken, J.A., Debruyne, F.M., Ru, N., and Isaacs, W.B. DD3: a new prostate-specific gene, highly overexpressed in prostate cancer. Cancer Res., 59: 5975-5979,1999.
3. Black, D.L. Mechanisms of alternative pre-messenger RNA splicing. Annu. Rev. Biochem, 2003,72:291-336.
4. Yelin, R, Dahary, D., Sorek, R, Levanon, E.Y., Goldstein, O., Shoshan, A., Diber, A., Biton, S., Tamir, Y., Khosravi, R, Nemzer,S., Pinner, E., Walach, S., Bernstein, J., Savitsky, K., and Rotman, G. Widespread occurrence of antisense transcription in the human genome. Nat. BiotechnoL, 21: 379-386, 2003.
5. de Kok, J.B., Verhaegh, G.W., Roelofs, R.W., Hessels, D., Kiemeney, L.A., Aalders, T.W., Swinkels, D.W., and Schalken, J.A. PCA3, a very sensitive and specific marker for to detect prostate tumors. Cancer Res., 62: 2695-2698, 2002.
6. Hessels, D., Klein Gunnewiek, J., Oort, I., Karthaus, H.F.M., van Leenders, G.J.L., van Balken, B., Kiemeney, LA., Witjes, J.A., and Schalken, J.A. PCA3-based molecular urine analysis for the diagnosis of prostate cancer. Eur. Urol., 2003, in press*.*

## Claims

1. An in vitro method for diagnosing or prognosing prostate cancer in a patient, comprising:
(i) amplifying a spliced prostate cancer specific PCA3 RNA which lacks at least intron 3, between exon 3 and exon 4, from a urine sample of a patient using a pair of primers; and
(ii) detecting an amplification product derived therefrom with a probe which is specific for an exon 3-exon 4 junction of PCA3,
wherein said amplification product is associated with the presence of prostate cancer or predisposition thereto in said patient.

2. The method of claim 1, wherein the spliced prostate cancer specific PCA3 RNA also lacks
(i) intron 1 between exon 1 and exon 2; or
(ii) intron 2 between exon 2 and exon 3.

3. The method of claim 1 or 2, wherein said PCA3 RNA is intron-less.

4. The method of any one of claims 1 to 3, wherein said probe is a hybridizing probe.

5. The method of claim 4, wherein said probe is detectably labelled.

6. A diagnostic kit for detecting the presence of spliced prostate cancer specific PCA3 RNA which lacks at least intron 3, between exon 3 and exon 4, in a urine sample of a patient, comprising:
(i) at least one pair of primers which is designed to enable amplification of said PCA3 RNA, wherein one primer is specific for an exon 3-exon 4 junction of PCA3; and
(ii) a probe which is specific for the exon 3-exon 4 junction of said PCA3.

7. A diagnostic kit for detecting the presence of spliced prostate cancer specific PCA3 RNA which lacks intron 3, between exon 3 and exon 4, in a urine sample of a patient, comprising:
(i) at least one pair of primers which is designed to enable amplification of said PCA3 RNA across an exon 3-exon 4 junction of PCA3; and
(ii) a probe which is specific for the exon 3-exon 4 junction of said PCA3.

## Patentansprüche

1. In vitro-Verfahren zur Diagnose und Prognose von Prostatakrebs in einem Patienten, das umfasst:
(i) das Amplifizieren einer gespleißten Prostatakrebs-spezifischen PCA3-RNA, der mindestens Intron 3 zwischen Exon 3 und Exon 4 fehlt, von einer Urinprobe eines Patienten unter Verwendung eines Primerpaares; und
(ii) das Nachweisen eines Amplifikationsprodukts, das davon abgeleitet ist, mit einer Sonde, die spezifisch für eine Exon 3 - Exon 4-Verknüpfung von PCA3 ist,
wobei das Amplifikationsprodukt mit dem Vorliegen von Prostatakrebs oder der Veranlagung dafür in diesem Patienten in Zusammenhang steht.

2. Verfahren nach Anspruch 1, wobei der gespleißten Prostatakrebs-spezifischen PCA3-RNA auch fehlt:
(i) Intron 1 zwischen Exon 1 und Exon 2, oder
(ii) Intron 2 zwischen Exon 2 und Exon 3.

3. Verfahren nach Anspruch 1 oder 2, wobei die PCA3-RNA intronlos ist.

4. Verfahren nach einem der Ansprüche 1 bis 3, wobei die Sonde eine Hybridisierungssonde ist.

5. Verfahren nach Anspruch 4, wobei die Sonde nachweisbar markiert ist.

6. Diagnose-Kit zum Nachweis des Vorliegens einer gespleißten Prostatakrebs-spezifischen PCA3-RNA, der mindestens Intron 3 zwischen Exon 3 und Exon 4 fehlt, in einer Urinprobe eines Patienten, das umfasst:
(i) mindestens ein Primerpaar, das so konzipiert ist, dass es die Amplifikation der PCA3-RNA ermöglicht, wobei ein Primer spezifisch ist für eine Exon 3 - Exon 4-Verknüpfung von PCA3; und
(ii) eine Sonde, die spezifisch ist für die Exon 3 - Exon 4-Verknüpfung des PCA3.

7. Diagnose-Kit zum Nachweis des Vorliegens einer gespleißten Prostatakrebs-spezifischen PCA3-RNA, der Intron 3 zwischen Exon 3 und Exon 4 fehlt, in einer Urinprobe eines Patienten, das umfasst:
(i) mindestens ein Primerpaar, das so konzipiert ist, dass es die Amplifikation der PCA3-RNA über eine Exon 3 - Exon 4-Verknüpfung von PCA3 hinweg ermöglicht; und
(ii) eine Sonde, die spezifisch ist für die Exon 3 - Exon 4-Verknüpfung des PCA3.

## Revendications

1. Procédé *in vitro* pour établir le diagnostic ou le pronostic du cancer de la prostate chez un patient, consistant à :
(i) amplifier un ARN de PCA3 spécifique du cancer de la prostate épissé qui est dépourvu d'au moins l'intron 3, entre l'exon 3 et l'exon 4, à partir d'un échantillon d'urine d'un patient en utilisant une paire d'amorces; et
(ii) détecter un produit d'amplification dérivé de celui-ci avec une sonde qui est spécifique pour une jonction exon 3-exon 4 de PCA3,
dans lequel ledit produit d'amplification est associé à la présence du cancer de la prostate ou à une prédisposition à celui-ci chez ledit patient.

2. Procédé selon la revendication 1, dans lequel l'ARN de PCA3 spécifique du cancer de la prostate épissé est également dépourvu
(i) de l'intron 1 entre l'exon 1 et l'exon 2 ; ou
(ii) de l'intron 2 entre l'exon 2 et l'exon 3.

3. Procédé selon la revendication 1 ou 2, dans lequel ledit ARN de PCA3 est dépourvu d'introns.

4. Procédé selon l'une quelconque des revendications 1 à 3, dans lequel ladite sonde est une sonde d'hybridation.

5. Procédé selon la revendication 4, dans lequel ladite sonde est marquée de manière détectable.

6. Kit de diagnostic pour détecter la présence d'un ARN de PCA3 spécifique du cancer de la prostate épissé qui est dépourvu d'au moins l'intron 3, entre l'exon 3 et l'exon 4, dans un échantillon d'urine d'un patient, comprenant :
(i) au moins une paire d'amorces qui est conçue pour permettre l'amplification dudit ARN de PCA3, où une amorce est spécifique pour une jonction exon 3-exon 4 de PCA3 ; et
(ii) une sonde qui est spécifique pour la jonction exon 3-exon 4 dudit PCA3.

7. Kit de diagnostic pour détecter la présence d'un ARN de PCA3 spécifique du cancer de la prostate épissé qui est dépourvu de l'intron 3, entre l'exon 3 et l'exon 4, dans un échantillon d'urine d'un patient, comprenant :
(i) au moins une paire d'amorces qui est conçue pour permettre l'amplification dudit ARN de PCA3 sur une jonction exon 3-exon 4 de PCA3 ; et
(ii) une sonde qui est spécifique pour la jonction exon 3-exon 4 dudit PCA3.
